# EUROPEAN PATENT SPECIFICATION

(11) **EP 1 964 838 B1**
(45) Date of publication and mention of the grant of the patent: **30.12.2009**
(21) Application number: 07004341.9
(22) Date of filing: 02.03.2007
(51) Int. Cl.: C07D 333/36

(54) **Process for the resolution of homocysteine-thiolactone**
Verfahren zur Auflösung von Homocystein-Thiolacton
Procédé de résolution d'homocystéine-thiolactone

(43) Date of publication of application: 03.09.2008
(73) Proprietor: EDMOND PHARMA S.R.L., 20157 Milano (IT)
(72) Inventor: Nicola, Massimo, 27100 Pavia (IT); Gatti, Pier Andrea, 27010 San Genesio ed Uniti (IT); Zacche, Matteo, 20010 Vanzago (IT)
(74) Representative: Minoja, Fabrizio

(56) References cited:
- JP-A- 2000 351 776
- JP-A- 2001 199 980

## Description

### FIELD OF THE INVENTION

The present invention relates to the preparation of enantiomerically pure 2-amino-4-mercaptobutyric acid 1,4-thiolactone.

### DESCRIPTION

2-Amino-4-mercaptobutyric acid 1,4-thiolactone, commonly referred to as homocysteine-γ-thiolactone (hereinafter indicated as HCT) I: 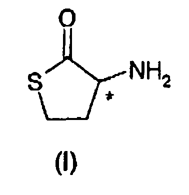 contains a chiral carbon atom, denoted with a *, and thus exists in the form of two distinct enantiomers, the R- and the S-enantiomer. The racemate is commercially available and is usually sold in the form of its hydrochloride salt.

A first known method for the preparation of enantiomerically pure HCT comprises the demethylation of enantiomerically pure methionine, to obtain enantiomerically pure homocysteine and then closing the lactone ring, thus obtaining enantiomerically pure HCT. This method is costly and difficult to carry out on an industrial scale.

Another method for the preparation of enantiomerically pure HCT is disclosed in JP 2001-199980 and JP 2000-351776, wherein racemic HCT is reacted with an enantiomerically pure acid to form two diastereomeric amides; the diastereoisomers are then separated and the amide is hydrolysed to obtain the desired enantiomer of HCT. Also this method presents many drawbacks, because the separation is performed in three steps and the cleavage of an amide is a difficult reaction. Moreover, it uses costly reagents and yields are low.

There is therefore the need for an improved process for the resolution of HCT enantiomers.

### DESCRIPTION OF THE INVENTION

It has now been found that enantiomerically pure homocysteine-y-thiolactone can be prepared through optical resolution of racemic homocysteine-γ-thiolactone 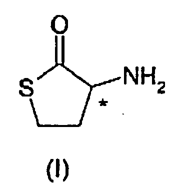 with a chiral organic acid wherein one isomer is recovered as a diastereomeric salt with the organic acid and the other isomer remaining in the mother liquor is submitted to racemisation with a catalytic amount of an aromatic aldehyde and submitted again to optical resolution with the same chiral organic acid.

In greater detail, the invention comprises two main embodiments: a first one (referred to as method 1 in the examples), wherein the diastereomeric salt remaining in the mother liquor is first isolated and then racemised with a catalytic amount of an aromatic aldehyde so as to provide racemic homocysteine-γ-thiolactone which is submitted again to optical resolution and a second, more advantageous one (referred to as method 2 in the examples), wherein optical resolution is carried out in the presence of a catalytic amount of an aromatic aldehyde, so that one out of the two diastereomeric salts precipitates and the other one remaining in the mother liquor racemises. In the following, the expression "mother liquor" denotes the solution wherefrom the less soluble diastereoisomer precipitates.

In greater detail, the first embodiment comprises the following steps:
a) preparing a solution of racemic HCT in a suitable solvent;
b) adding the solution with an enantiomerically pure chiral organic acid;
c) optionally heating the solution and then cooling it until complete precipitation of the less soluble diastereomeric salt;
d) separating the precipitated diastereomeric salt from the solution and liberating enantiomerically pure HCT 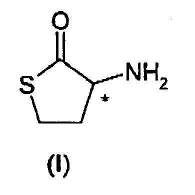
e) adding the solution from step d) with HCI until complete precipitation of HCT hydrochloride enriched in the enantiomer which remained in solution;
f) recovering the precipitate and treating it with a carboxylic acid, preferably acetic acid, in the presence of an aromatic aldehyde so as to precipitate racemic HCT hydrochloride salt;
g) isolating racemic HCT hydrochloride salt;
h) dissolving the salt in the same organic solvent as step a) and adding a base so as to liberate racemic HCT;
i) repeating steps b)-d) and optionally e) - h) one or more times.

The solvent used for the preparation of the solution of racemic HCT of step a) is a solvent or a mixture of solvents selected from water, alcohols, such as methanol, ethanol, propanol, isopropanol, or another suitable organic solvent, for example acetone or tetrahydrofuran. Enantiomerically pure chiral organic acids suitable for carrying out step b) are, for example, dibenzoyltartaric acid, ditoluoyltartaric acid, mandelic acid, tartaric acid, camphorsulfonic acid, ascorbic acid, malic acid, pyrogluthammic acid, aspartic acid, chinic acid, the most preferred being ditoluoyltartaric acid and mandelic acid. When heating of the solution is necessary in order to precipitate the less soluble diastereomeric salt, the temperature varies according to the solvent and can be increased up to the reflux temperature. Enantiomerically pure HCT recovered from step d) is preferably converted into the hydrochloride salt by dissolution in acetone and addition of hydrogen chloride, until complete precipitation of HCT hydrochloride.

The starting solution of HCT can be prepared by dissolving commercially available racemic HCT hydrochloride in one or more of the above-mentioned solvents and adding an organic or inorganic base, such as triethylamine or hydroxides of alkaline metals. The same base is also used for step h).

As indicated above, preferred resolving agents are ditoluoyltartaric acid and mandelic acid, since they allow to obtain yields ranging from 70 to 80 percent based on the theoretical amount of the desired enantiomer.

When using ditoluoyltartaric acid, the process is carried out by dissolving HCT hydrochloride in methanol and adding ditoluoyltartaric acid at room temperature. The resulting solution is added with a stoichiometric amount of triethylamine, so that triethylamine hydrochloride and the most soluble diastereomeric salt remain in the solution, while the less soluble diastereomeric salt precipitates. The precipitate is collected by filtration and further purified by treatment in acetone at room temperature. After filtration, HCT dibenzoyltartrate is suspended again in acetone and hydrochloric acid is added until complete precipitation of enantiomerically pure HCT hydrochloride.

When using instead mandelic acid as the resolving agent, HCT hydrochloride is suspended in acetone and a stoichiometric amount of triethylamine is added. Triethylamine hydrochloride is filtered off and mandelic acid is added to the filtrate (which contains HCT as free base), until complete precipitation of the less soluble diastereomeric salt. The precipitate is collected by filtration, then suspended again in acetone and acidified with hydrochloric acid until precipitation of enantiomerically pure HCT hydrochloride.

The aromatic aldehyde used in step f) is selected from salicylaldehyde, m-hydroxy-benzaldehyde, p-hydroxy-benzaldehyde, o-anisaldehyde, m- anisaldehyde, p- anisaldehyde, salicylaldehyde being preferred; heating is required in order to accomplish racemisation. After 1-12 hours, depending on the temperature, the solution is cooled until complete precipitation of racemic HCT hydrochloride, which is collected by filtration and washed with acetone, then submitted again to the resolution process.

This first embodiment can be represented as in the following Scheme 1: 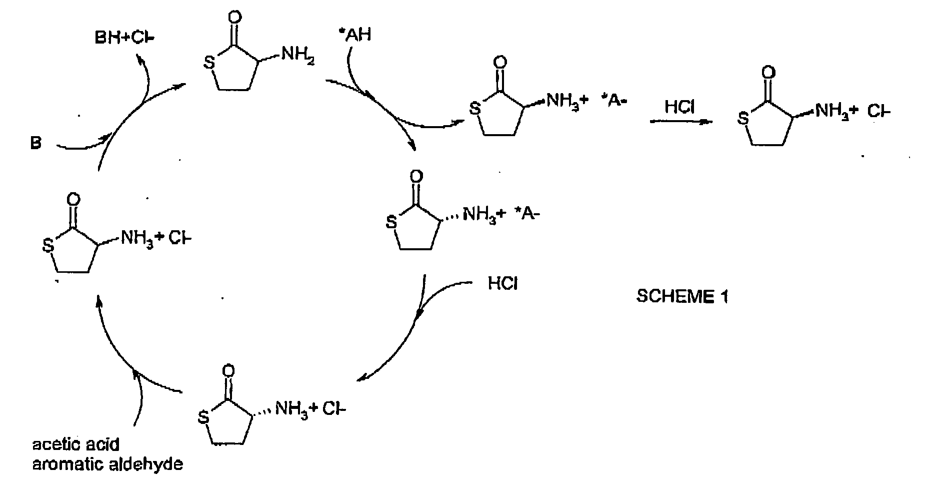 in which **B** denotes a base and ***AH** denotes a chiral acid (resolving agent).

The second embodiment of the invention comprises the following steps:
a) preparing a solution of racemic HCT in a suitable solvent;
b) adding the solution with an enantiomerically pure chiral organic acid and a catalytic amount of an aromatic aldehyde;
c) heating the solution and then cooling it until precipitation of the less soluble diastereomeric salt of HCT with the chiral organic acid is complete;
d) separating the precipitated diastereomeric salt from the solution and liberating enantiomerically pure 2-amino-4-mercaptobenzobutyic acid 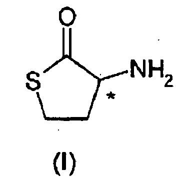
e) optionally adding HCl to the solution from step d) and recovering racemic HCT hydrochloride to be submitted to a further resolution cycle.

The starting solution can be prepared in the same way and with the same solvents as the first embodiment and steps c) - d) are also carried out as described above.

This second embodiment has the remarkable advantage that the most soluble diastereomeric salt continuously racemise in the solution, while the less soluble one precipitates subtracting from the racemisation reaction, which is an equilibrium. Thus, at any point of the process only the racemate is present in the solution. The process continues until an equilibrium is reached between the solubility of the precipitated salt (regulated by its Kₛₚ) and the kinetics of the racemisation reaction (regulated by its kinetic constant). Enantiomerically pure HCT is preferably isolated in the form of the hydrochloride salt by suspension in acetone and treatment with HCl.

This embodiment allows to obtain yields of enantiomerically pure HCT hydrochloride higher than the theoretical amount, usually not lower than 14Q%, calculated on the amount of the desired enantiomer in the racemate. This is possible because the undesired enantiomer is converted *in situ* into the desired one.

After acidification and filtration, racemic HCT hydrochloride can be recovered from the mother liquor and can be submitted to a further resolution cycle, thus increasing yields further.

This second preferred embodiment can be represented as in the following scheme 2, wherein the aromatic aldehyde is salicylaldehyde:

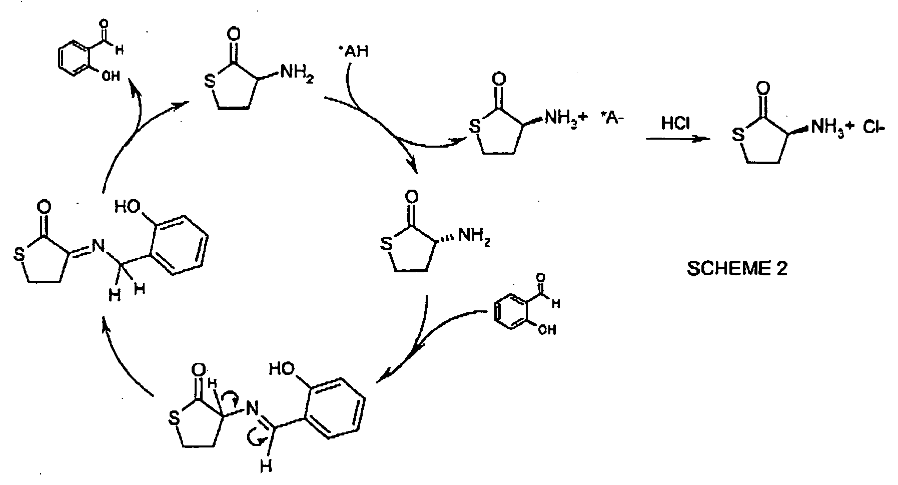

The invention will be now illustrated in greater detail by means of the following examples.

### EXAMPLES

### Example 1 - Resolution (method 1)

R,S-HCT hydrochloride (154 g) and acetone (2500 mL) are charged into a reactor, added with triethylamine (139 mL) and stirred for an hour. Triethylamine hydrochloride is filtered off and the light yellow filtrate is charged again into the reactor with L-(+)-mandelic acid (114 g). A solid soon begins to precipitate; the suspension is cooled to 10°C for one hour and then filtered. S-HCT mandelate (118 g dry weight) is obtained, while the mother liquors are saved for the racemisation process.

S-HCT mandelate is charged into the reactor with acetone (1500 mL) and hydrochloric acid 37% (44 mL) is added to the stirred suspension. Stirring is maintained for one hour at 15°C and the solid is filtered and washed with acetone. S-HCT hydrochloride is obtained (60.5 g dry weight, 78.5% yield), with an [α]_{D} = +21.9°.

### Example 2 - Resolution (method 1)

R,S-HCT hydrochloride (154 g), methanol (3000 mL) and (-)-ditoluoyl-L-tartaric acid (145 g) are charged into a reactor and the mixture is stirred until complete dissolution, then triethylamine (139 mL) is added dropwise at 20°C. Stirring is maintained at the same temperature for one hour, then the precipitated solid is filtered and washed with methanol; the mother liquors are saved for the racemisation process. The precipitate is instead charged again into the reactor with acetone (1500 mL) and stirred for one hour at room temperature, then filtered, obtaining L-HCT ditoluoyltartrate (120 g dry weight).

S-HCT ditoluoyltartrate is suspended again in acetone (1500 mL) and 37% hydrochloric acid (40 mL) is added to the stirred suspension. Stirring is maintained for one hour at 15°C and the resulting product is filtered and washed with acetone, obtaining 55.5 g of L-TCT hydrochloride (dry weight, 72% yield), with an [α]_{D} = +22.1 °.

### Example 3 - Racemisation (method 1)

The mother liquors from Example 1 are acidified to pH 1 with 37% hydrochloric acid and then concentrated to dryness under vacuum. The residue is dissolved in acetic acid (150 mL), added with salicylaldehyde (2 mL) and the solution is heated to 50°C for 5 hours, then cooled to 5°C for 2 hours. The resulting solid is filtered and washed thoroughly with acetone, obtaining racemic R,S-HCT hydrochloride (65.5 g dry weight).

### Example 4 - Resolution (method 2)

R,S-HCT hydrochloride (250 g) is suspended in acetone (1250 mL) into a reactor and added with triethylamine (226.5 mL) under stirring. Stirring is continued for at least one hour, then triethylamine hydrochloride is filtered off and the light yellow filtrate is charged again into the reactor with L-(+)-mandelic acid (250 g) and salicylaldehyde (25 g). The solution turns persistent yellow, indicating imine formation, then a solid begins to precipitate. The suspension is stirred at room temperature for 5 hours, then the solid is collected by filtration and washed with acetone; the mother liquors are saved for the recovery of racemic HCT. The wet solid is suspended again in acetone (2500 mL) and added under stirring with 37% hydrochloric acid (109 mL). Stirring is maintained for one hour at 5°C and the solid is filtered and washed with acetone, obtaining 175 g of S-HCT hydrochloride (dry weight, 140% yield based on the desired enantiomer), with an [α]_{D} = +22.5°.

### Example 5 - Recovery of starting material (Method 2)

The mother liquors from Example 4 are acidified to pH 1 with hydrochloric acid and cooled to 5°C for 4 hours. The solid is collected by filtration and dried under vacuum, yielding 25 g of racemic R,S-HCT hydrochloride.

## Claims

1. A process for the preparation of enantiomerically pure homocysteine-γ-thiolactone comprising optical resolution of racemic homocysteine-γ-thiolactone (HCT) 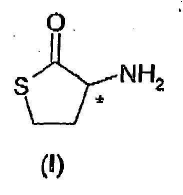 with a chiral organic acid wherein one isomer is recovered as diastereomeric salt with the organic acid and the other isomer remaining in the mother liquor is submitted to racemisation with a catalytic amount of an aromatic aldehyde selected from salicylaldehyde, m-hydroxy-benzaldehyde, p-hydroxy-benzaldehyde, o-anisaldehyde, m- anisaldehyde, p- anisaldehyde and submitted again to optical resolution with the same chiral organic acid.

2. A process a according to claim 1 wherein the diastereomeric salt remaining in the mother liquor is first isolated and then racemised with a catalytic amount of the aromatic aldehyde so as to provide racemic homocysteine-γ-thiolactone which is submitted again to optical resolution.

3. A process according to claim 2 which comprises the following steps:
a) preparing a solution of racemic HCT in a suitable solvent;
b) adding the solution with an enantiomerically pure chiral organic acid;
c) optionally heating the solution and then cooling it until complete precipitation of the less soluble diastereomeric salt;
d) separating the precipitated diastereomeric salt from the solution and liberating enantiomerically pure HCT 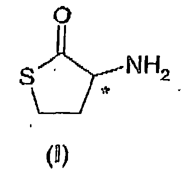
e) adding the solution from step d) with HCl until complete precipitation HCT hydrochloride salt enriched in the enantiomer which remained in the solution;
f) recovering the precipitate and treating it with acetic acid in the presence of the aromatic aldehyde so as to precipitate racemic HCT hydrochloride salt;
g) isolating racemic HCT hydrochloride salt;
h) dissolving the salt in the same organic solvent as step a) and adding a base so as to liberate racemic HCT;
i) repeating steps b)-d) and optionally e) - h) one or more times.

4. A process according to claim 1 wherein optical resolution is carried out in the presence of a catalytic amount of an aromatic aldehyde, so that one out of the two diastereomeric salts precipitates and the other one remaining in the solution racemises.

5. A process according to claim 4 which comprises the following steps:
a) preparing a solution of racemic HCT in a suitable solvent;
b) adding the solution with an enantiomerically pure chiral organic acid and a catalytic amount of an aromatic aldehyde;
c) heating the solution and then cooling it until complete precipitation of the less soluble diastereomeric salt of HCT with the chiral organic acid;
d) separating the precipitated diastereomeric salt from the solution and liberating enantiomerically pure 2-amino-4-mercaptobenzobutyric acid 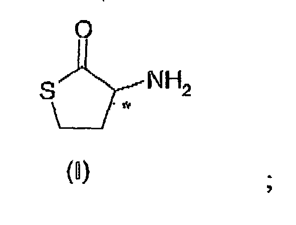
e) optionally adding HCl to the solution from step d) and recovering racemic HCT.

6. A process according to any one of claims 1 to 5 wherein the enantiomerically pure organic acid is selected from dibenzoyltartaric acid, ditoluoyltartaric acid, mandelic acid, tartaric acid, camphorsulfonic acid, ascorbic acid, malic acid, pyrogluthammic acid, aspartic acid, chinic acid, the most preferred being ditoluoyltartaric acid and mandelic acid.

7. A process according to claim 6 wherein the enantiomerically pure organic acid is ditoluoyltartaric acid or mandelic acid.

8. A process according to claim 7 wherein the aromatic aldehyde is salicylaldehyde.

## Patentansprüche

1. Eine Verfahren zur Herstellung von enantiomerenreinem Homocystein-γ-thiolacton, das die optische Auflösung von racemischem Homocystein-γ-thiolacton (HCT) 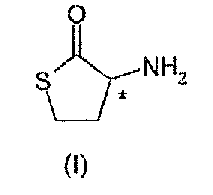 mit einer chiralen organischen Säure umfasst, wobei ein Isomer als ein diastereomeres Salz mit der organischen Säure wiedergewonnen wird, und das andere, in der Mutterlauge verbleibende isomer einer Racemisierung mit einer katalytischen Menge eines aromatischen Aldehyds unterzogen wird, das ausgewählt wird aus Salicylaldehyd, m-Hydroxybenzaldehyd, p-Hydroxybenzaldehyd, o-Anisaldehyd, m-Anisaldehyd, p-Anisaldehyd, und wieder einer optischen Auflösung mit derselben chiralen organischen Säure unterworfen wird.

2. Ein Verfahren nach Anspruch 1, wobei das in der Mutterlauge verbleibende diastereomere Salz zunächst isoliert wird und dann mit einer katalytischen Menge des aromatischen Aldehyds racemisiert wird, um racemisches Homocystein-γ-thiolacton bereitzustellen, das wiederum einer optischen Auflösung unterworfen wird.

3. Ein Verfahren nach Anspruch 2, das die folgenden Schritte umfasst:
a) Herstellen einer Lösung von racemischem HCT in einem geeigneten Lösungsmittel;
b) Zusetzen einer enantiomerenreinen chiralen organischen Säure zu der Lösung;
c) gegebenenfalls Erhitzen der Lösung und anschließendes Abkühlen der Lösung bis zur vollständigen Ausfällung des schlechter löslichen diastereomeren Salzes;
d) Abtrennen des ausgefällten diastereomeren Salzes aus der Lösung und Freisetzung von enantiomerenreinem HCT 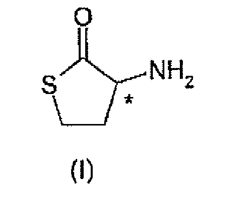
e) Zusetzen von HCl zu der Lösung aus Schritt d) bis zur vollständigen Ausfällung von HCT-Hydrochloridsalz, das mit dem in der Lösung verbliebenen Enantiomer angereichert ist;
f) Gewinnen des Niederschlages und Behandeln des Niederschlages mit Essigsäure in Anwesenheit des aromatischen Aldehyds, um so racemisches HCT-Hydrochloridsalz auszufällen;
g) Isolieren von racemischen HCT-Hydrochloridsalz;
h) Auflösen des Salzes in demselben Lösungsmittel wie in Schritt a) und Zusetzen einer Base, um racemisches HCT freizusetzen;
i) ein- oder mehrmaliges Wiederholen der Schritte b) - d), und gegebenenfalls e) - h).

4. Ein Verfahren nach Anspruch 1, wobei die optische Auflösung in Anwesenheit einer katalytische Menge eines aromatischen Aldehyds erfolgt, so dass eines der beiden diastereomeren Salze ausfällt und das andere, in der Lösung verbleibende Salz racemisiert.

5. Ein Verfahren nach Anspruch 4, das die folgenden Schritte umfasst:
a) Herstellen einer Lösung von racemischem HCT in einem geeigneten Lösungsmittel;
b) Zusetzen einer enantiomerenreinen chiralen organischen Säure und einer katalytische Menge eines aromatischen Aldehyds zu der Lösung;
c) Erhitzen der Lösung und anschließendes Abkühlen der Lösung bis zur vollständigen Ausfällung des schlechter löslichen diastereomeren Salzes von HCT mit der chiralen organischen Säure;
d) Abtrennen des ausgefällten diastereomeren Salzes aus der Lösung und Freisetzung von enantiomerenreiner 2-Amino-4-mercaptobenzobuttersäure 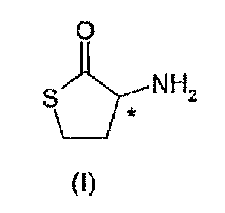
e) gegebenenfalls Zusetzen von HCl zu der Lösung aus Schritt d) und Wiedergewinnen von racemischen HCT.

6. Ein Verfahren nach einem der Ansprüche 1 bis 5, wobei die enantiomerenreine organische Säure aus Dibenzoylweinsäure, Ditoluoylweinsäure, Mandelsäure, Weinsäure, Camphersulfonsäure, Ascorbinsäure, Apfelsäure, Pyroglutaminsäure, Asparaginsäure, Chinasäure ausgewählt wird, wobei die bevorzugtesten Ditoluoylweinsäure und Mandelsäure sind.

7. Ein Verfahren nach Anspruch 6, wobei die enantiomerenreine organische Säure Ditoluoylweinsäure oder Mendelsäure ist.

8. Ein Verfahren nach Anspruch 7, wobei das aromatische Aldehyd Salicylaldehyd ist.

## Revendications

1. Procédé pour la préparation d'homocystéine-γ-thiolactone énantiomériquement pure comprenant la résolution optique de l'homocystéine-γ-thiolactone (HCT) racémique 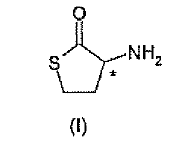 avec un acide organique chiral, dans lequel un isomère est récupéré en tant que sel diastéréoisomérique avec l'acide organique et l'autre isomère restant dans la liqueur-mère est soumis à racémisation avec une quantité catalytique d'un aldéhyde aromatique choisi parmi le salicylaldéhyde, le m-hydroxy-benzaldéhyde, le p-hydroxy-benzaldéhyde, l'o-anisaldéhyde, le m-anisaldéhyde, le p-anisaldéhyde et soumis à nouveau à une résolution optique avec le même acide organique chiral.

2. Procédé selon la revendication 1, dans lequel le sel diastéréoisomérique restant dans la liqueur-mère est d'abord isolé et puis racémisé avec une quantité catalytique de l'aldéhyde aromatique afin de fournir l'homocystéine-γ-thiolactone racémique qui est soumise à nouveau à la résolution optique.

3. Procédé selon la revendication 2 qui comprend les étapes suivantes:
a) la préparation d'une solution de HCT racémique dans un solvant approprié;
b) l'ajout de la solution avec un acide organique chiral énantiomériquement pur;
c) optionnellement, le chauffage de la solution et le refroidissement de celle-ci jusqu'à la précipitation complète du sel diastéréoisomérique le moins soluble;
d) la séparation du sel diastéréoisomérique précipité à partir de la solution et la libération de HCT énantiomériquement pur 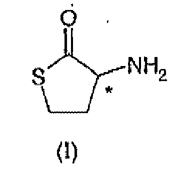
e) l'ajout de la solution de l'étape d) avec HCl jusqu'à la précipitation complète du sel de chlorhydrate de HCT enrichi en l'énantiomère qui est resté dans la solution;
f) la récupération du précipité et le traitement de celui-ci avec de l'acide acétique en présence de l'aldéhyde aromatique afin de précipiter le sel de chlorhydrate de HCT racémique;
g) l'isolement du sel de chlorhydrate de HCT racémique;
h) la dissolution du sel dans le même solvant organique que l'étape a) et l'ajout d'une base afin de libérer HCT racémique;
i) la répétition des étapes b)-d) et optionnellement e)-h) une ou plusieurs fois.

4. Procédé selon la revendication 1 dans lequel la résolution optique est effectuée en présence d'une quantité catalytique d'un aldéhyde aromatique, de sorte qu'un sur les deux sels diastéréoisomériques précipite et l'autre restant dans la solution se racémise.

5. Procédé selon la revendication 4 qui comprend les étapes suivantes:
a) la préparation d'une solution de HCT racémique dans un solvant approprié;
b) l'ajout de la solution avec un acide organique chiral énantiomériquement pur et une quantité catalytique d'un aldéhyde aromatique;
c) le chauffage de la solution et puis le refroidissement de celle-ci jusqu'à la précipitation complète du sel diastéréoisomérique de HCT le moins soluble avec l'acide organique chiral ;
d) la séparation du sel diastéréoisomérique précipité à partir de la solution et la libération de l'acide 2 amino-4-mercaptobenzobutyrique énantiomériquement pur 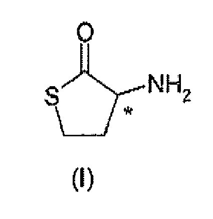
e) optionnellement, l'ajout de HCl à la solution de l'étape d) et la récupétion de HCT racémique.

6. Procédé selon l'une quelconque de revendications 1 à 5, dans lequel l'acide organique énantiomériquement pur est sélectionné parmi l'acide dibenzoyltartrique, l'acide ditoluoyltartrique, l'acide mandélique, l'acide tartrique, l'acide camphorsulfonique, l'acide ascorbique, l'acide malique, l'acide pyroglutamique, l'acide aspartique, l'acide chinique, le préféré étant l'acide ditoluoyltartrique et l'acide mandélique.

7. Procédé selon la revendication 6, dans lequel l'acide organique énantiomériquement pur est l'acide ditoluoyltartrique ou l'acide mandélique.

8. Procédé selon la revendication 7, dans lequel l'aldéhyde aromatique est le salicylaldéhyde.
